Europäisches Patentamt

European Patent Office

Office européen des brevets

(19)

(11) Veröffentlichungsnummer : **0 129 710
B1**

(12) **EUROPÄISCHE PATENTSCHRIFT**

(45) Veröffentlichungstag der Patentschrift :
06.07.88

(51) Int. Cl.⁴ : **C 11 D   1/28, C 07 C139/14**

(21) Anmeldenummer : 84105793.8

(22) Anmeldetag : 21.05.84

(54) **Verfahren zur Gewinnung farbstabiler hellfarbiger wässriger Salzpasten von waschaktiven alpha-Sulfofettsäureestern.**

(30) Priorität : 30.05.83 DE 3319591

(43) Veröffentlichungstag der Anmeldung :
02.01.85 Patentblatt 85/01

(45) Bekanntmachung des Hinweises auf die Patenterteilung : 06.07.88 Patentblatt 88/27

(84) Benannte Vertragsstaaten :
AT BE CH DE FR IT LI NL SE

(56) Entgegenhaltungen :
EP-A- 0 054 724
DE-A- 1 443 995
DE-A- 2 555 076
US-A- 3 354 187
Die Akte enthält technische Angaben, die nach dem Eingang der Anmeldung eingereicht wurden und die nicht in dieser Patentschrift enthalten sind.

(73) Patentinhaber : Henkel Kommanditgesellschaft auf Aktien
Postfach 1100 Henkelstrasse 67
D-4000 Düsseldorf-Holthausen (DE)

(72) Erfinder : Klötzer, Dietrich, Dr.
Am Rittersberg 8
D-4000 Düsseldorf 13 (DE)
Erfinder : Linde, Karl-Heinz
Erlenweg 8
D-4018 Langenfeld (DE)
Erfinder : Ojust, Klaus
Am Langen Weiher 51
D-4000 Düsseldorf 13 (DE)

## Beschreibung

Die US-A-2 195 187 beschreibt waschaktive Substanzen auf Basis von α-Sulfofettsäureestern. Sie werden durch Sulfonierung niederer Alkylester gesättigter höherer Fettsäuren mit Schwefeltrioxid erhalten. Die als Ausgangsmaterial eingesetzten niederen Fettsäurealkylester werden durch Umesterung hydrierter Fette oder Öle mit einwertigen niederen Alkanolen, insbesonder Methanol, bzw. durch Verseifung der Fette oder Öle und anschließende Veresterung mit den Alkanolen hergestellt.

Die DE-B-1 246 718 beschreibt ein Verfahren zur Herstellung dieser Verbindungsklasse, bei dem Fettsäuren und Fettsäureester, die 6-28 C-Atome in Fettsäurerest aufweisen und außer dem α-ständigen C-Atom des Fettsäurerestes keine weiteren sulfonierbaren bzw. sulfatierbaren Gruppen aufweisen und eine Iodzahl unterhalb 5 besitzen mit einem Schwefeltrioxid-Inertgas-Gemisch sulfoniert werden, woraufhin das Umsetzungsprodukt neutralisiert wird. Ein mit alternativen Verfahrensmerkmalen, letztlich aber mit gleichen Mitteln arbeitendes Parallelverfahren zur Herstellung dieser Verbindungen ist in der DE-B-1 248 645 beschrieben.

Eine der Hauptschwierigkeiten des hier betroffenen Gebiets liegt in der Farbinstabilität des Fettsäure bzw. Fettsäureester enthaltenden Ausgangsmaterials im Sulfonierungsschritt. Regelmäßig werden dunkelgefärbte bis braunschwarze Rohprodukte erhalten, die für eine spätere Anwendung in Wasch- und Reinigungsmitteln zu hellfarbigen Produkten aufzuarbeiten sind. Die Farbe der Sulfonierungsrohprodukte ist zwar in bestimmten Ausmaß von der Reinheit der Ausgangsmaterialien und den Arbeitsbedingungen der Sulfonierungsstufe abhängig, gleichwohl wird die technische Verwertung der hier als Ausgangsmaterialien dienenden natürlichen Öle und Fette durch die folgende Gesetzmäßigkeit behindert : Je höher die Umsatzausbeute in der Sulfonierungsstufe (der Sulfonierungsgrad) getrieben wird, um so dunkler gefärbt ist das Reaktionsprodukt und um so größer werden die Schwierigkeiten, hellfarbige Endprodukte zu gewinnen.

Stets ist also als abschließender Verfahrensschritt eine Bleiche der Rohsulfonsäure-Derivate nötig. Der Stand der Technik hat hier insbesondere zwei Verfahrenstypen entwickelt : Die saure Bleiche mit Wasserstoffperoxid — siehe hierzu beispielsweise DE-C-1 179 931 — oder eine Kombinationsbleiche, bei der sich an eine zunächst saure Wasserstoffperoxidbleiche die Neutralisierung des sulfonierten und teilgebleichten Gutes anschließt, woraufhin erneut mit Wasserstoffperoxid oder besser mit Hypochlorit eine abschließende Bleichstufe erfolgt — siehe hierzu beispielsweise die DE-B-1 234 709.

Besondere Schwierigkeiten zum Problem der Verfärbung treten auf, wenn die Sulfonierung auf Umsatzausbeuten über 90 % oder gar auf Sulfonierungsgrade über 95 % getrieben werden soll.

Die DE-A-1 443 995 beschäftigt sich ausführlich mit den hier entstehenden Problemen. Das Schwefeltrioxid hat nach den Angaben dieser Druckschrift auf gesättigte, von alkoholischen Hydroxylgruppen freie Fettsäureester eine stark zersetzende Wirkung, die bei der Herstellung hochsulfonierter Produkte mit einem Sulfonierungsgrad von wenigstens 90 %, vorzugsweise wenigstens 94 %, und insbesondere wenigstens 96 % unvermeidbar zu tiefdunkel verfärbten Sulfonierungsrohprodukten führt.

Die Erhöhung des Ausmaßes der Sulfonierung in solche Bereiche ist aber nicht nur aus wirtschaftlichen Gründen interessant. Weiterführende Gesetzmäßigkeiten fordern derart hohe Sulfonierungsgrade. So gilt : Estersulfonate mit vergleichsweise niedrigen Sulfonierungsgraden führen zu Schwierigkeiten bei der üblichen Herstellung von Waschmittelkompositionen durch Zerstäuben. Beim Verarbeiten solcher Estersulfonate treten erfahrungsgemäß hohe Pluming-Werte auf. Weiterhin steht der Sulfonierungsgrad von Estersulfonaten in direktem Zusammenhang mit unerwünschten Nebenprodukten, die sich bei dieser Reaktion bilden, den α-Sulfofettsäuren. Diese nach der Neutralisation als Di-Natrium-Salz vorliegenden Verbindungen sind nur schlecht wasserlöslich und daher als Waschrohstoffe untauglich. Eine Erhöhung des Sulfonierungsgrades von 90 % auf 96 % in den Estersulfonaten bewirkt eine Abnahme dieser unerwünschten Nebenprodukte von beispielsweise 25 % auf 16 %.

Die Lehre der DE-A-1 443 995 schlägt zur Einschränkung der Verfärbung neben der Einhaltung bestimmter Temperaturen in der Sulfonierungsreaktion vor, so viel Wasser in das Sulfonierungsprodukt einzubringen, daß sich aus dem vorhandenen überschüssigen Schwefeltrioxid und dem Wasser eine Schwefelsäure bildet, deren Konzentration bei Beginn der nachfolgenden Bleiche im Bereich von 100-20 Gewichtsprozent $H_2SO_4$ liegt. Für die großtechnische Verwirklichung werden hierdurch jedoch neue Schwierigkeiten erzeugt, die eine beträchtliche Gefahrenquelle darstellen können. Die Viskosität des Sulfonierungsrohprodukts wird im stark sauren Bereich schon durch geringste Wassermengen schwerwiegend beeinflußt.

Schon der Zusatz von 2 Gewichtsprozent Wasserstoffperoxid in Form einer 35 gewichtsprozentigen Lösung — und den damit zwangsläufig eingeführten Wassermengen — zum rohen Sulfonierungsprodukt mit der Kettenlänge C-16/C-18 führt zu einem steilen Viskositätsanstieg. Hierdurch entsteht im kontinuierlichen technischen Verfahren die Gefahr der Verstopfung von Rohrleitungen. Besonders kritisch ist dieser Viskositätsanstieg schon im Bereich des Zusatzes von 1,8-2,5 Gewichtsprozent Wasserstoffperoxid, berechnet auf die Rohsulfonsäure.

Als bisher bestes Bleichverfahren für Rohestersulfonate ist die Verwendung von Wasserstoffperoxid im stark sauren Bereich (pH = 0) beschrieben. Der Bleicheffekt ist hier besonders stark ausgeprägt. Es werden jedoch die zuvor geschilderten Gefahren des plötzlichen Viskositätsanstieges ausgelöst. Bei hoch-sulfonierten Estersulfonaten reichen selbst 2 Gewichtsprozent Wasserstoffperoxid nicht aus, die gewünschten niedrigen Klett-Farbzahlen einzustellen. Nach Neutralisation der Rohsulfonsäure muß daher mit Natriumhypochlorit nachgebleicht werden. Eine Verringerung der Bleichmittelmenge und gleichzeitige Erhöhung der Bleichzeit bei der sauren Bleichung mit Wasserstoffperoxid führt zu ungünstigeren Farben. Im Zusammenhang damit besteht eine Reihe weiterer Schwierigkeiten : Durch den hohen Viskositätsanstieg bei Zusatz von beispielsweise 2 Gewichtsprozent Wasserstoffperoxid zur Rohsulfonsäure ist es nicht möglich, bei der Neutralisation höhere Pastenkonzentrationen als ca. 28 Gewichtsprozent Waschaktivsubstanz (WAS) herzustellen. Bei dieser Bleichmethode treten zudem Probleme mit der Schaumbildung auf, die technisch nur schwer beherrschbar sind. Insbesondere führt der in die Rohsulfonsäure eingerührte Schaum zu weiterem Viskositätsanstieg.

Die vielgestaltigen und in unterschiedlichen Stufen des Gesamtverfahrens auftretenden Schwierigkeiten führen nach bisherigem Wissen zu einem aufgezwungenen Kompromiß zwischen Sulfonierung und Bleichung. Die in der Praxis optimal einstellbaren Sulfonierungsgrade liegen bei ca. 90 %.

Die DE-A-3 047 897 beschreibt eine Möglichkeit, wie die Verfärbung des Sulfonierungsendproduktes durch einen zusätzlichen Reinigungsschritt am zu sulfonierenden Fettsäureesterausgangsmaterial verringert werden kann. Bei dieser Verfahrensmodifikation bringt man in die Sulfonierungsstufe eine Fettsäureesterfraktion ein, aus der die begleitenden Fettsäureglyceride bis auf einen Restgehalt von höchstens etwa 1 Gewichtsprozent, vorzugsweise auf nicht mehr als etwa 0,3 Gewichtsprozent — jeweils bezogen auf das zu sulfonierende Material — entfernt worden sind. Die Beseitigung dieser « begleitenden Fettsäureglyceride » aus den Estern der Fettsäuren bzw. Fettsäuregemische natürlichen Ursprungs mit einwertigen Alkoholen erfordert eine wenigstens zweifache Destillation der zu sulfonierenden Fettsäureesterfraktion.

Die Lehre der vorliegenden Erfindung geht von der Aufgabe aus, ein einfaches und sicher reproduzierbares Bleichverfahren für α-Sulfofettsäureester bzw. ihre Salze, insbesondere aus Fetten und Ölen pflanzlichen und/oder tierischen Ursprungs zu entwickeln, das hohe Umsatzausbeuten in der Sulfonierungsstufe zuläßt, die vorzugsweise oberhalb von 95 Gewichtsprozent und insbesondere oberhalb von 97 Gewichtsprozent liegen, gleichwohl aber ohne zwingende Redestillation der intermediär gewonnenen Fettsäureesterfraktion zu hellfarbigen, farbstabilen Produkten führt. Aufgabe der Erfindung ist es weiterhin, in diesem Bleichverfahren die Gewinnung hochkonzentrierter wässriger Salzpasten zu ermöglichen, deren Gehalt an WAS beispielsweise bis zu 60 Gewichtsprozent reichen kann. Das erfindungsgemäße Verfahren will dabei schließlich einen Bleichweg aufweisen, der in seiner großtechnischen Anwendung frei ist von den beschriebenen Sicherheitsrisiken der Wasserstoffperoxidbleiche an den sauren α-Sulfofettsäureestern, gleichwohl aber Wasserstoffperoxid als einen substantiellen Bestandteil des Bleichverfahrens einsetzt.

Die erfindungsgemäße Lösung dieses Komplexes mehrerer Teilaufgaben wurde in einer Kombinationsbleiche gefunden, die mit den an sich bekannten Bleichmitteln Wasserstoffperoxid und Hypochlorit arbeitet, dabei jedoch die Reihenfolge ihrer Anwendung gegenüber den vorbekannten Vorschlägen umkehrt und darüber hinaus in allen Stufen dieser Bleichbehandlung als zu bleichendes Gut die wässrigen Salzaufschlämmungen der WAS vorsieht.

Gegenstand der Erfindung ist dementsprechend ein Verfahren zur Gewinnung farbstabiler, hellfarbiger wässriger Salzpasten waschaktiver Ester von α-Sulfofettsäuren mit einwertigen $C_1$-$C_8$-Alkoholen (α-Sulfofettsäureester), insbesondere aus Fetten und Ölen pflanzlichen und/oder tierischen Ursprungs, und ihrer Abmischungen mit waschaktiven Alkylbenzosulfonaten durch eine Kombinationsbleiche des dunkelfarbigen rohen Ausgangsmaterials mit Wasserstoffperoxid und Alkalihypochlorit, wobei dieses Verfahren dadurch gekennzeichnet ist, daß man das dunkelfarbige Ausgangsmaterial durch Salzbildung an der α-Sulfosäuregruppe zur Estersulfonatpaste umwandelt, die Estersulfonatpaste in wässrigem Medium im pH-Bereich von etwa 7 bis 11, vorzugsweise von 7,5 bis 10, zunächst mit Hypochlorit einer Vorbleiche unterwirft und anschließend erforderlichenfalls noch vorhandenes Hypochlorit beseitigt, woraufhin man die Salzpaste im pH-Bereich von etwa 3,5 bis unterhalb 7, vorzugsweise im pH-Bereich von etwa 3,5 bis unterhalb 7, vorzugsweise im pH-Bereich von etwa 4,0 bis 6,5 mit Wasserstoffperoxid bzw. Wasserstoffperoxid liefernden Verbindungen nachbleicht.

Auch das erfindungsgemäße Verfahren arbeitet also mit einer Kombinationsbleiche, bei der der angestrebte Bleicheffekt anteilsweise durch Hypochlorit und anteilsweise durch Wasserstoffperoxid erzielt wird. Entscheidend ist hier jedoch insbesondere die Kombination der folgenden Maßnahmen : Es wird die bestimmte Reihenfolge in der Anwendung der Bleichmittel derart gewählt, daß zunächst dem Hypochlorit der überwiegende Teil der zu leistenden Bleicharbeit zufällt und erst in einer abschließenden Stufe mit Wasserstoffperoxid nachgebleicht wird und wobei weiterhin beide Bleichmittel — insbesondere also gerade das Wasserstoffperoxid — an den in der Salzform vorliegenden α-Sulfofettsäureestern zum Einsatz kommen. Der pH-Wert einer wässrigen Aufschlämmung des Mononatriumsalzes von α-Sulfofettsäureestern liegt bekanntlich im Bereich von etwa 3,5, so daß sich hieraus ableitet, daß beide Bleichstufen im pH-Bereich oberhalb dieses Grenzwertes stattfinden.

Im einzelnen gilt zum erfindungsgemäßen Verfahren und zu seinen Teilstufen das Folgende :

Zur Vorbleichung mit Hypochlorit wird zunächst das Sulfonierungsrohprodukt durch Salzbildung an der α-Sulfosäuregruppe zur Estersulfonatpaste umgewandelt. Eingesetzt werden hierzu üblicherweise Alkalimetallhydroxide und/oder Amine. Bevorzugtes salzbildendes Neutralisationsmittel ist Natriumhydroxid. Hypochlorit wird in Form einer NaOCl-Lösung zugesetzt, wobei etwa 5-20 gewichtsprozentige, insbesondere etwa 10-15 gewichtsprozentige NaOCl-Lösungen besonders geeignet sind. Die Menge an eingesetztem NaOCl liegt üblicherweise bei bis zu etwa 4 Gewichtsprozent — bezogen auf WAS und jeweils berechnet als 100 %ige Substanz. Üblicherweise wird mit NaOCl-Mengen von etwa 0,1-3 Gewichtsprozent gearbeitet — wiederum bezogen auf WAS und wie zuvor berechnet. Als Temperaturbereich dieser Hypochloritvorbleiche haben sich insbesondere etwa 50-75 °C, vorzugsweise Temperaturen im Bereich von etwa 55-70 °C als geeignet erwiesen. Die Bleichdauer liegt üblicherweise im Bereich von etwa 1-10 Stunden, insbesondere im Bereich von etwa 1-3 Stunden. Dabei gilt im einzelnen das Folgende :

Die im pH-Wert korrigierte wässrige Estersulfonatpaste wird mit einer beispielsweise 12-13 %igen NaOCl-Lösung versetzt. Der Zusatz kann einstufig oder mehrstufig erfolgen. Danach wird der während der Bleichreaktion sich einstellende Farbaufhellungseffekt und der zum jeweils gleichen Zeitpunkt vorherrschende Chlorgehalt des Reaktionsgemisches einer stetigen Kontrolle unterworfen. Bei fortschreitender Bleichreaktionsdauer kann eine Verlangsamung der Produktaufhellung meßtechnisch z. B. anhand photometrisch ermittelter Farbzahlen, beobachtet werden. Es tritt im Verlauf der Reaktion eine Verlangsamung der Farbverbesserung ein, die parallel mit einer Abnahme des Verbrauchs an Bleichmittel verläuft. Bezüglich der einzustellenden Farbwerte der Paste gilt, daß bei dieser Vorbleiche mit Hypochlorit jeweils ein Bleichoptimum durchlaufen wird, das durch die hellste Farbe der Paste gekennzeichnet ist. Nach Durchlaufen dieses Bleichoptimums tritt eine mehr oder weniger starke Farbumkehr bzw. erneute Farbverschlechterung ein. In der bevorzugten Ausführungsform der Erfindung wird die Hypochloritvorbleiche etwa zum Zeitpunkt dieses Bleichoptimums abgebrochen.

An diese erste Bleichstufe schließt sich erforderlichenfalls eine Zwischenbehandlung an, in der die im Reaktionsgemisch noch verbliebenen Hypochloritanteile beseitigt werden. Dieses kann schon alleine durch pH-Absenkung in den schwach sauren Bereich erfolgen, es kann aber auch — je nach der noch vorliegenden Hypochlorit-Restmenge — zu deren beschleunigter Entfernung ein Reduktionsmittel zugesetzt werden. Geeignet ist hierzu insbesondere die Zugabe geringer Mengen an Aldehyden, insbesondere Formaldehyd. Es können aber auch reduzierende Salze, beispielsweise Alkalimetallsulfite oder Alkalimetallthiosulfate hierzu eingesetzt werden. Als Verfahrensbedingungen für diese Zwischenbehandlung mit Reduktionsmitteln können die unveränderten Werte aus der Hypochloritvorbleiche beibehalten werden, insbesondere kann also hierbei im Temperaturbereich von etwa 50-75 °C, bevorzugt im Bereich von etwa 55-70 °C, und in dem pH-Bereich der Hypochloritbleiche gearbeitet werden. Die Zeitdauer dieser Zwischenstufe beträgt im allgemeinen 0,5-5 Stunden, insbesondere etwa 1-2 Stunden.

Falls noch erforderlich, wird nach Abschluß dieser Reduktionsstufe die wässrige Estersulfonatpaste auf niedrigere pH-Werte des angegebenen Bereichs, z. B. auf einen pH-Werte im Bereich von etwa 4,0-6,5 eingestellt. Hierzu können anorganische oder organische Säuren verwendet werden. Eine bevorzugte Säure zur pH-Regulierung ist Schwefelsäure. Diese pH-Herabsetzung dient der Vorbereitung der sich anschließenden Nachbleichung mittels Wasserstoffperoxid.

Wasserstoffperoxid kann beispielsweise in Mengen bis zu 2,5 Gewichtsprozent, vorzugsweise im Bereich von etwa 1-2 Gewichtsprozent — jeweils berechnet als 100 %iges $H_2O_2$ und bezogen auf WAS — eingesetzt werden. Geeignet sind hier beispielsweise etwa 20-70 gewichtsprozentige wässrige $H_2O_2$-Lösungen. Auch diese Stufe der Nachbleichung mit Wasserstoffperoxid erfolgt bevorzugt bei Temperaturen oberhalb von etwa 50 °C, insbesondere im Temperaturbereich von etwa 55-70 °C. Durch diese Nachbleichung mit $H_2O_2$ tritt eine weitere Farbaufhellung der Estersulfonatpaste ein. Im allgemeinen ist allerdings die Farbaufhellung dieser Stufe im Vergleich mit der Hypochloritbleiche nur noch beschränkt. Hervorzuheben ist jedoch, daß die erfindungsgemäß erhaltenen Estersulfonatpasten wesentlich farbstabiler sind als entsprechende nur mit Natriumhypochlorit gebleichte Pasten. Auch die Dauer der Wasserstoffperoxidbleiche kann — je nach der Menge des eingesetzten $H_2O_2$ — bis zu einigen Stunden betragen und dabei beispielsweise bis zu etwa 20 Stunden in Anspruch nehmen.

Ein entscheidender Vorteil der erfindungsgemäßen Nachbleichung mit Wasserstoffperoxid an wässrigen pastenförmigen Suspensionen der Salze der WAS — statt an den noch nicht neutralisierten sauren Sulfonierungsprodukten gemäß den Angaben des Standes der Technik — liegt in der Möglichkeit, vergleichsweise hohe WAS-Konzentrationen in der wässrigen Paste vorliegen zu haben und behandeln zu können, ohne daß gleichzeitig hiermit ins Gewicht fallende technische Sicherheitsrisiken verbunden sind. So ist es beispielsweise möglich, mit WAS-Gehalten in der wässrigen Paste im Bereich von 20-30 Gewichtsprozent zu arbeiten, ohne daß es des Zusatzes von Fließhilfsmitteln bedürfte und ohne daß Verstopfungen des Gesamtsystems durch plötzlichen Viskositätsanstieg zu befürchten sind. Werden Fließhilfsmittel bzw. Viskositätsregler mitverwendet, so kann die Konzentration der WAS in den zu bleichenden Pasten beispielsweise bis auf 60 Gewichtsprozent angehoben werden.

Als Fließhilfsmittel bzw. Viskositätsregler eignen sich u. a. höhermolekulare Polyglykolethergruppen enthaltende Verbindungen wie Polyethylenglykole mit einem Molekulargewicht von 600 bis 6 000, Polypropylenglykole mit einem Molekulargewicht von 250 bis 4 000, Anlagerungsprodukte von 20 bis 80 Mol Ethylenoxid an aliphatische Alkohole mit 10 bis 20 Kohlenstoffatomen und Anlagerungsprodukte von 20 bis 60 Mol Ethylenoxid an Alkylphenole mit 6 bis 12 Kohlenstoffatomen in der Alkylgruppe. Diese Verbindungen werden den wässrigen Pasten in Mengen von maximal 10 Gewichtsprozent, vorzugsweise 0,1 bis 5 Gewichtsprozent und insbesondere 0,5 bis 3 Gewichtsprozent, bezogen auf die WAS-Konzentrate, zugesetzt.

Weiterhin können als Fließhilfsmittel bzw. Viskositätsregler in α-Stellung durch Halogen-, Cyan- oder Sulfogruppen substituierte $C_1$- bis $C_6$-Alkylmonocarbonsäuren ; deren Salze oder deren Ester mit $C_1$- bis $C_6$-Alkanolen, insbesondere mit Ethanol oder Methanol ; in α-Stellung durch Halogen-, Cyan- oder Sulfogruppen substituierte Alkylendicarbonsäuren ; deren Salze oder deren Ester mit $C_1$- bis $C_6$-Alkanolen, insbesondere mit Ethanol oder Methanol ; Nitrilotriessigsäure und deren Salze und Etheralkohole mit 2 bis 4 Alkylenglykolethereinheiten und einer Methoxy- oder Ethoxygruppe eingesetzt werden. Diese Verbindungen werden in Mengen von 1 bis 15 Gewichtsprozent, vorzugsweise 7-12 Gewichtsprozent, bezogen auf den WAS-Gehalt, den wässrigen Pasten zugegeben.

Als Fließhilfsmittel bzw. Viskositätsregler eignen sich ferner Alkohole mit 8 bis 40 Kohlenstoffatomen, die zusätzlich eine oder mehrere Hydroxylgruppen als Substituenten aufweisen können, und Anlagerungsprodukte von bis zu 20 Mol Ethylenoxid und/oder Propylenoxid an solche Alkohole. Diese Verbindungen werden den wässrigen Pasten in Mengen von 1 bis 15 Gewichtsprozent, bezogen auf den WAS-Gehalt, zugesetzt.

Schließlich können auch N-Chlorderivate von Arylsulfonsäureamid-Natriumverbindungen, beispielsweise N-Chlor-p-toluolsulfonsäureamid-Natrium und N-Chlorbenzolsulfonsäureamid-Natrium als Fließhilfsmittel bzw. Viskositätsregler eingesetzt werden. Diese Verbindungen werden in Mengen von 0,5 bis 10 Gewichtsprozent, bezogen auf das Konzentrat, eingesetzt.

Mit dem erfindungsgemäßen Verfahren können ohne eine Redestillation der aus natürlichen Ausgangsmaterialien gewonnenen Fettsäurealkylester Farbwerte und Farbbeständigkeiten erhalten werden, die denen der besten bisher bekannten Verfahren — die eine Aufhellungsbehandlung an entsprechend beschriebenen Sulfierungsneutralprodukten zum Inhalt haben — wenigstens vergleichbar sind, wenn nach identischer Meßmethodik mit gleichen WAS-Konzentrationen in Küvetten gleicher Größe bzw. mit gleicher Schichtdicke gemessen wird.

Das erfindungsgemäße Bleichverfahren bettet sich damit in ein Mehrstufenverfahren ein, das von natürlichen Ausgangsmaterialien, insbesonder pflanzlichen und/oder tierischen Ölen ausgehend zu hochwertigen Tensidkomponenten führt. Im einzelnen gelten zu diesen zahlreichen Stufen die Angaben des zitierten Standes der Technik, die hier nur kurz wiederholt seien :

Die als Ausgangsmaterialien eingesetzten Öle und/oder Fette können Pflanzen, Land- oder Wassertieren entstammen. Ihre Fettsäurereste liegen überwiegend innerhalb des C-Zahlbereichs von 8-18, wobei sowohl Fette mit bevorzugt 10-14 Kohlenstoffatomen in den Fettsäureresten als solche mit bevorzugt 16-18 Kohlenstoffatomen in den Fettsäureresten gleichermaßen geeignet sind. Sulfatierbare oder sulfonierbare Gruppen, die — abgesehen vom α-ständigen Wasserstoffatom des Fettsäurerestes — in diesen Fettsäuren bzw. deren Estern nicht vorhanden sein sollen, sind beispielsweise Doppelbindungen oder alkoholische Hydroxylgruppen. Ursprünglich vorliegende Doppelbindungen können durch Hydrierung beseitigt werden. Es ist dabei möglich, diese Hydrierung am Fett- bzw. Öl-Ausgangsmaterial vorzunehmen. Bevorzugt wird jedoch der nach der Glyceridspaltung erhaltene Fettsäurealkylester hydrierend gesättigt. Diese Hydrierung erfolgt in an sich bekannter Weise. Die Produkte der Hydrierung sollen Iodzahlen unter 2, vorzugsweise unter 1 und insbesondere im Bereich von 0,5 oder darunter aufweisen. Bevorzugte Ausgangsmaterialien für die anschließende Sulfonierung sind Talgfettsäuremethylester und Palmölettsäuremethylester.

Das Fettsäureesterausgangsmaterial wird in an sich bekannter Weise z. B. bei Temperaturen von 70-130 °C in einem Sulfierungsreaktor mit einem Gemisch von gasförmigen Schwefeltrioxid und Inertgas während eines Zeitraums von 10 sek. bis 120 min. sulfoniert. Der Sulfonierungsgrad beträgt vorzugsweise mehr als 90 %, insbesondere mehr als 92 % und in der Regel mehr als 94 %. Sulfonierungsgrade von 95 % und darüber sind besonders bevorzugt.

Die anschließende Bleiche erfolgt in der zuvor eingehend beschriebenen erfindungsgemäßen Ausgestaltung. Das gebleichte Gut kann als solches direkt dem Slurry zur Gewinnung der trockenen Waschmittel zugegeben werden. Es kann aber auch zur neutralen Paste neutralisiert und in dieser Form gelagert und/oder verschickt werden.

In einer Ausführungsform der Erfindung werden die α-Sulfofettsäureester bzw. ihre Vorstufen zusammen mit anderen Tensidkomponenten, insbesondere mit Alkylbenzolsulfonaten (ABS) eingesetzt. Hierbei können beliebige Mischungsverhältnisse zwischen ABS und α-Sulfofettsäureestern zur Verwendung kommen, die also beispielsweise im Bereich von 5-95 Gewichtsprozent zu 95-5 Gewichtsprozent liegen.

Die folgenden Beispiele erläutern die Erfindung :

Beispiele

Die Herstellung der in den nachfolgenden Beispielen 1 bis 9 als Ausgangsmaterial verwendeten Estersulfonatpasten erfolgte durch konventionelle Sulfonierung von Fettsäuremethylestern. Der Säureanteil der Methylester bestand im wesentlichen aus gesättigten $C_{16}$- und $C_{18}$-Fettsäuren. Die Sulfonierungsreaktion wurde in einer dreistufigen Kaskadenanlage bei 90-95 °C mit einem Schwefeltrioxid-/Luftgemisch ($SO_3$-Gehalt 2-8 Volumenprozent) bei einem Schwefeltrioxidüberschuß von 15-25 Molprozent durchgeführt. Das rohe Sulfonierungsprodukt wurde mit verdünnter Natronlauge neutralisiert.

Alle Farbmessungen erfolgten fotometrisch nach Klett an 5 gewichtsprozentigen WAS-Lösungen in einer 4 cm Küvette mit Blaufilter bei 400-465 nm.

Beispiel 1

Als Ausgangsmaterial diente eine Estersulfonatpaste mit einem WAS-Gehalt von 32 Gewichtsprozent und einer Klett-Farbzahl von 975. Der Sulfonierungsgrad betrug 97 %. Dieses Material war durch Sulfonierung eines Talgfettsäuremethylesters erhalten worden, in dem der Säureanteil zu 40 Gewichtsprozent aus $C_{16}$-Fettsäure und zu 60 Gewichtsprozent aus $C_{18}$-Fettsäure bestand.

In einem heizbaren Reaktionsgefäß wurden 5 000 kg Ausgangsmaterial (1 600 kg WAS) unter kräftigem Rühren bei 60-70 °C mit 295,6 kg einer 13 gewichtsprozentigen Natriumhypochloritlösung (38,4 kg NaOCl) versetzt. Das Gemisch, das einen pH-Wert von 9,5 besaß, wurde 3 Stunden lang bei 60-70 °C gerührt. Dabei wurde nach 1,5 Stunden Rührzeit eine Klett-Farbzahl von 320 und nach 3 Stunden Rührzeit eine Klettfarbzahl von 298 ermittelt.

Durch Zugabe von 20 gewichtsprozentiger Schwefelsäure wurde nun der pH-Wert auf 4 abgesenkt und das Gemisch 2 Stunden lang bei 60-70 °C weitergerührt, bevor 53,4 kg einer 30 gewichtsprozentigen Wasserstoffperoxidlösung (16 kg $H_2O_2$) zugegeben wurden. Das Gemisch wurde bei 60-70 °C 20 Stunden lang gerührt. Dabei wurde nach 3 Stunden Rührzeit eine Klettfarbzahl von 265 und nach 20 Stunden Rührzeit eine Klett-Farbzahl von 240 gemessen. Abschließend wurde der pH-Wert der Estersulfonatpaste mit verdünnter Natronlauge auf 7 eingestellt.

Beispiel 2

Beispiel 1 wurde wiederholt mit der Abhänderung, daß die Wasserstoffperoxidbleiche mit der doppelten Menge Bleichmittel, d. h. mit 106,8 kg einer 30 gewichtsprozentigen Wasserstoffperoxidlösung (32 kg $H_2O_2$), durchgeführt wurde. Nach 3 Stunden Rührzeit wurde eine Klett-Farbzahl von 230 und nach 20 Stunden Rührzeit eine Klett-Farbzahl von 210 beobachtet.

Beispiel 3

In einem heizbaren Reaktionsgefäß wurden 5 000 kg des Ausgangsmaterials aus Beispiel 1 (1 600 kg WAS) unter kräftigem Rühren mit 591,2 kg einer 13 gewichtsprozentigen Natriumhypochloritlösung (76,8 kg NaOCl) versetzt. Danach hatte das Gemisch einen pH-Wert von 10,5 ; es wurde 4 Stunden lang bei 60-70 °C gerührt. Zwei Stunden nach der Hypochloritzugabe wurde eine Klett-Farbzahl von 230 und nach weiteren 2 Stunden eine Klett-Farbzahl von 183 gemessen. Durch Zugabe von 20 gewichtsprozentiger Schwefelsäure wurde dann ein pH-Wert von 4 eingestellt und das Gemisch zur Zersetzung des nicht verbrauchten Hypochlorits 2,5 Stunden lang bei 60-70 °C gerührt. Danach wurden 54,3 kg einer 30 gewichtsprozentigen Wasserstoffperoxidlösung (16 kg $H_2O_2$) zugegeben und das Gemisch 3 Stunden lang bei 60-70 °C weitergerührt. Die Estersulfonatpaste, die nun eine Klett-Farbzahl von 155 zeigte, wurde mit verdünnter Natronlauge auf einen pH-Wert von 7 eingestellt.

Beispiel 4

Als Ausgangsmaterial diente eine Estersulfonatpaste mit einem WAS-Gehalt von 32 Gewichtsprozent und einer Klett-Farbzahl von 960. Der Sulfonierungsgrad betrug 95 %. Dieses Material war durch Sulfonierung eines Talgfettsäuremethylesters erhalten worden, in dem der Säureanteil zu 50 Gewichtsprozent aus $C_{16}$-Fettsäure und zu 50 Gewichtsprozent aus $C_{18}$-Fettsäure bestand.

Analog Beispiel 1 wurden 5 000 kg Estersulfonatpaste (1 600 kg WAS) bei etwa 65 °C mit 123,1 kg einer 13 gewichtsprozentigen Natriumhypochloritlösung (16 kg NaOCl) versetzt. Das Gemisch hatte einen pH-Wert von 8 ; es wurde 2 Stunden lang bei 60-70 °C gerührt, wobei die Klett-Farbzahl auf 450 zurückging.

Durch Zugabe von 20 gewichtsprozentiger Schwefelsäure wurde dann der pH-Wert auf 4 gesenkt und die Paste 30 Minuten lang bei etwa 65 °C weitergerührt, bevor 106,8 kg einer 30 gewichtsprozentigen Wasserstoffperoxidlösung (32 kg $H_2O_2$) zugegeben wurden. Das Gemisch wurde 20 Stunden lang bei etwa 65 °C gerührt. Dabei wurde nach 3 Stunden eine Klett-Farbzahl von 370 und nach 20 Stunden eine Klett-Farbzahl von 330 gemessen. Abschließend wurde der pH-Wert der Estersulfonatpaste mit 20 gewichtsprozentiger Natronlauge auf 7,5 eingestellt.

Beispiel 5

Analog Beispiel 1 wurden 5 000 kg des Ausgangsmaterials aus Beispiel 4 (1 600 kg WAS) mit 246,2 kg einer 13 gewichtsprozentigen Natriumhypochloritlösung (32 kg NaOCl) versetzt. Das Gemisch, das nun einen pH-Wert von 9,5 hatte, wurde 2 Stunden lang bei etwa 65 °C gerührt, wobei die Klett-Farbzahl auf 310 zurückging.

Durch Zugabe von 20 gewichtsprozentiger Schwefelsäure wurde der pH-Wert auf 4 gesenkt und die Paste 1 Stunde lang bei etwa 65 °C weitergerührt, bevor 53,4 kg einer 30 gewichtsprozentigen Wasserstoffperoxidlösung (16 kg $H_2O_2$) zugegeben wurden. Das Gemisch wurde 20 Stunden lang bei etwa 65 °C gerührt. Nach 3 Stunden Rührzeit wurde eine Klett-Farbzahl von 270 und nach 20 Stunden eine Klett-Farbzahl von 240 ermittelt. Abschließend wurde der pH-Wert der Estersulfonatpaste mit verdünnter Natronlauge auf 6,5 eingestellt.

Beispiel 6

Beispiel 5 wurde mit der Abänderung wiederholt, daß die Wasserstoffperoxidbleiche mit der doppelten Menge Bleichmittel, d. h. mit 106,8 kg einer 30 gewichtsprozentigen Wasserstoffperoxidlösung (32 kg $H_2O_2$), durchgeführt wurde. Nach 3 Stunden Rührzeit wurde eine Klett-Farbzahl von 230 und nach 20 Stunden eine Klett-Farbzahl von 205 gemessen.

Beispiel 7

Analog Beispiel 1 wurden 5 000 kg des Ausgangsmaterials aus Beispiel 4 (1 600 kg WAS) mit 369,3 kg einer 13 gewichtsprozentigen Natriumhypochloritlösung (48 kg NaOCl) versetzt. Das Gemisch hatte danach einen pH-Wert von 10 ; es wurde 4 Stunden lang bei etwa 65 °C gerührt. Nach 2 Stunden Rührzeit hatte das Gemisch eine Klett-Farbzahl von 385, nach 4 Stunden Rührzeit eine Klett-Farbzahl von 325.

Nach dem Absenken des pH-Wertes mit Hilfe von 20 gewichtsprozentiger Schwefelsäure wurde das Gemisch 90 Minuten lang bei etwa 65 °C weitergerührt. Anschließend wurden 53,4 kg einer 30 gewichtsprozentigen Wasserstoffperoxidlösung (16 kg $H_2O_2$) zugegeben und das Gemisch 20 Stunden lang bei etwa 65 °C gerührt. Nach 3 Stunden Rührzeit wurde eine Klett-Farbzahl von 250 und nach 20 Stunden eine Klett-Farbzahl von 220 ermittelt. Das Gemisch wurde abschließend mit 20 gewichtsprozentiger Natronlauge auf einen pH-Wert von 7,0 eingestellt.

Beispiel 8

Beispiel 7 wurde mit der Abänderung wiederholt, daß die Wasserstoffperoxidbleiche mit der doppelten Menge Bleichmittel, d. h. mit 106,8 kg einer 30 gewichtsprozentigen Wasserstoffperoxidlösung (32 kg $H_2O_2$), durchgeführt wurde. Drei Stunden nach der Bleichmittelzugabe zeigte die Estersulfonatpaste eine Klett-Farbzahl von 210. Nach 20 Stunden Rührzeit war die Klett-Farbzahl auf 170 zurückgegangen.

Beispiel 9

Als Ausgangsmaterial diente eine Estersulfonatpaste mit einem WAS-Gehalt von 30 Gewichtsprozent und einer Klett-Farbzahl von 950. Der Sulfonierungsgrad betrug 94 %. Dieses Material war durch Sulfonierung eines Talgfettsäuremethylesters erhalten worden, in dem der Säureanteil zu 40 Gewichtsprozent aus $C_{16}$-Fettsäure und zu 60 Gewichtsprozent aus $C_{18}$-Fettsäure bestand.

In einem heizbaren Reaktionsgefäß wurden 5 000 kg Ausgangsmaterial (1 500 kg WAS) unter kräftigem Rühren bei 65-70 °C mit 240 kg einer 12,5 gewichtsprozentigen Natriumhypochloritlösung (30 kg NaOCl) versetzt. Das Gemisch, das einen pH-Wert von 9,5 aufwies, wurde 2 Stunden lang bei 65-70 °C gerührt. Danach wurde eine Klett-Farbzahl von 280 beobachtet.

Anschließend wurden 50 kg einer 30 gewichtsprozentigen Formaldehydlösung (15 kg Formaldehyd) zugegeben und das Gemisch 2 Stunden lang bei 65-70 °C weitergerührt. Danach wurde der pH-Wert mittels 20 gewichtsprozentiger Schwefelsäure auf 6,0 eingestellt, bevor 50 kg einer 30 gewichtsprozentigen Wasserstoffperoxidlösung (60 kg $H_2O_2$) zugegeben wurden. Im Anschluß an diese Bleichmittelzugabe wurde das Gemisch 10 Stunden lang bei 65-70 °C gerührt. Danach zeigte die Paste eine Klett-Farbzahl von 252. Das Gemisch wurde zum Schluß mit 20 gewichtsprozentiger Natronlauge auf einen pH-Wert von 6,8 eingestellt.

Beispiel 10

Als Ausgangsmaterial diente eine Estersulfonatpaste mit einem WAS-Gehalt von 30 Gewichtsprozent und einer Klett-Farbzahl von 970. Der Sulfonierungsgrad betrug 96 %. Dieses Material war durch Sulfonierung eines Talgfettsäuremethylesters erhalten worden, in dem der Säureanteil zu 50 Gewichtsprozent aus $C_{16}$-Fettsäure und zu 50 % aus $C_{18}$-Fettsäure bestand.

In einem heizbaren Reaktionsgefäß wurden 5 000 kg Ausgangsmaterial (1 500 kg WAS) unter kräftigem Rühren bei 60 bis 70 °C mit 115,3 kg einer 13 gewichtsprozentigen Natriumhypochloritlösung (15 kg NaOCl) versetzt. Das Gemisch zeigte einen pH-Wert von 7,9. Nach einer Rührzeit von 30 min. wurde eine Klettzahl von 960 gemessen.

Nun wurden weitere 115,3 kg einer 13 gewichtsprozentigen Natriumhypochloritlösung (15 kg NaOCl) zugegeben. Danach wurde im Gemisch ein pH-Wert von 8,9 gemessen. Es wurde 90 Minuten lang bei 60 bis 70 °C weitergerührt. Danach wurde eine Klettzahl von 450 ermittelt.

Nach einer weiteren Zugabe von 115,3 kg 13 gewichtsprozentiger Natriumhypochloritlösung (15 kg NaOCl) zeigte das Gemisch einen pH-Wert von 9,8. Es wurde 150 Minuten lang bei 60 bis 70 °C weitergerührt. Die Klettzahl des Gemisches verminderte sich dabei auf 255.

Es wurden nun noch 55,7 kg 13 gewichtsprozentige Natriumhypochloritlösung (7,5 kg NaOCl) zugegeben, wodurch der pH-Wert des Gemisches auf 10,2 anstieg, und dann 15 1/2 Stunden lang bei 60 bis 70 °C gerührt. Am Ende der Hypochloritbleiche, d. h. nach einer Gesamtzugabe von 52,5 kg NaOCl und einer Gesamtrührzeit von 20 Stunden, war die Klettzahl der Estersulfonatpaste von anfangs 970 auf 200 zurückgegangen.

Der pH-Wert des Gemisches wurde nun mittels verdünnter Schwefelsäure auf 4,8 gesenkt und 50 kg einer 30 gewichtsprozentigen Wasserstoffperoxidlösung (15 kg $H_2O_2$) zugegeben. Das Ge-

misch wurde anschließend 20 Stunden lang bei 60 bis 70 °C gerührt. Danach zeigte das Gemisch eine Klettzahl von 173. Zum Schluß wurde der pH-Wert der Estersulfonatpaste mit 20 gewichtsprozentiger Natriumhydroxidlösung auf 7,5 eingestellt.

**Patentansprüche**

1. Verfahren zur Gewinnung farbstabiler, hellfarbiger wässriger Salzpasten waschaktiver Ester von $\alpha$-Sulfofettsäuren mit einwertigen $C_1$-$C_8$-Alkoholen ($\alpha$-Sulfofettsäureester), insbesondere aus Fetten und Ölen pflanzlichen und/oder tierischen Ursprungs, und ihrer Abmischungen mit waschaktiven Alkylbenzolsulfonaten durch eine Kombinationsbleiche des dunkelfarbigen rohen Ausgangsmaterials mit Wasserstoffperoxid und Alkalihypochlorit, dadurch gekennzeichnet, daß man das dunkelfarbige Ausgangsmaterial durch Salzbildung an der $\alpha$-Sulfosäuregruppe zur Estersulfonatpaste umwandelt, die Estersulfonatpaste in wässrigem Medium im pH-Bereich von etwa 7 bis 11, vorzugsweise von 7,5 bis 10, zunächst mit Hypochlorit einer Vorbleiche unterwirft und anschließend erforderlichenfalls noch vorhandenes Hypochlorit beseitigt, woraufhin man die Salzpaste im pH-Bereich von etwa 3,5 bis unterhalb 7, vorzugsweise im pH-Bereich von etwa 4,0 bis 6,5 mit Wasserstoffperoxid bzw. Wasserstoffperoxid liefernden Verbindungen nachbleibt.

2. Verfahren nach Anspruch 1, dadurch gekennzeichnet, daß die Hypochlorit-Vorbleiche etwa zum Zeitpunkt des Bleichoptimums abgebrochen wird.

3. Verfahren nach Ansprüchen 1 und 2, dadurch gekennzeichnet, daß die Hypochlorit-Vorbleiche mit bis zu etwa 4 Gew.-% NaOCl, vorzugsweise etwa 0,1 bis 3 Gew.-% NaOCl — jeweils bezogen auf Waschaktivsubstanz — durchgeführt wird, wobei das Hypochlorit dem zu bleichenden Gut bevorzugt mehrstufig zugesetzt wird.

4. Verfahren nach Ansprüchen 1-3, dadurch gekennzeichnet, daß die Beseitigung eines Hypochloritüberschusses nach Abschluß der ersten Bleichstufe durch pH-Absenkung in den schwach-sauren Bereich von etwa 3,5 bis unterhalb 7 und/oder durch Zusatz von Reduktionsmitteln erfolgt, wobei als Reduktionsmittel bevorzugt Formaldehyd, Alkalisulfite oder Alkalithiosulfate eingesetzt werden.

5. Verfahren nach Ansprüchen 1 bis 4, dadurch gekennzeichnet, daß zur pH-Einstellung bei der Peroxidbleiche Schwefelsäure eingesetzt wird.

6. Verfahren nach Ansprüchen 1-5, dadurch gekennzeichnet, daß das Wasserstoffperoxid in Mengen bis zu 2,5 Gewichtsprozent, vorzugsweise im Bereich von 1-2 Gewichtsprozent — jeweils berechnet als 100 %iges $H_2O_2$ und bezogen auf Waschaktivsubstanz — eingesetzt wird.

7. Verfahren nach Ansprüchen 1-6, dadurch gekennzeichnet, daß etwa 10-15 gewichtsprozentige NaOCl-Lösungen und ebenfalls etwa 20-70 gewichtsprozentige $H_2O_2$-Lösungen eingesetzt werden.

8. Verfahren nach Ansprüchen 1-7, dadurch gekennzeichnet, daß die Hypochlorit-Bleiche bei Temperaturen von etwa 50-75 °C, insbesondere im Bereich von 55-70 °C durchgeführt wird, wobei vorzugsweise mit einer Bleichdauer im Bereich von 1-10 Stunden, insbesondere im Bereich von 1-3 Stunden gearbeitet wird.

9. Verfahren nach Ansprüchen 1-8, dadurch gekennzeichnet, daß auch die Reduktionszwischenstufe und die nachfolgende Bleiche mit Wasserstoffperoxid bei Temperaturen oberhalb 50 °C, insbesondere im Bereich von 55-70 °C, durchgeführt werden.

10. Verfahren nach Ansprüchen 1-9, dadurch gekennzeichnet, daß in allen Verfahrensstufen bei Normaldruck gearbeitet wird.

11. Verfahren nach Ansprüchen 1-10, dadurch gekennzeichnet, daß mit wässrigen Salzpasten eines Gehalts an Alkalisalzen der $\alpha$-Sulfofettsäureester von etwa 20-60 Gewichtsprozent gearbeitet wird.

**Claims**

1. A process for the production of colour-stable, light-coloured aqueous salt pastes of washing-active esters of $\alpha$-sulfofatty acids with monohydric $C_1$-$C_8$ alcohols ($\alpha$-sulfofatty acid esters), more especially from fats and oils of vegetable and/or animal origin, and mixtures thereof with washing-active alkyl benzenesulfonates by subjecting the dark-coloured starting material to combined bleaching with hydrogen peroxide and alkali hypochlorite, characterized in that the dark-coloured starting material is transformed into the ester sulfonate paste by salt formation at the $\alpha$-sulfo acid group, the ester sulfonate paste is first subjected to preliminary bleaching with hypochlorite in aqueous medium at a pH value of from about 7 to 11 and preferably at a pH value of 7.5 to 10 and, if necessary, and hypochlorite still present is subsequently removed, after which the salt paste is afterbleached with hydrogen peroxide or with compounds yielding hydrogen peroxide at a pH value in the range from about 3.5 to below 7 and preferably at a pH value in the range from about 4.0 to 6.5.

2. A process as claimed in Claim 1, characterized in that the preliminary bleaching with hypochlorite is terminated at around the time when the bleaching effect is optimal.

3. A process as claimed in Claims 1 and 2, characterized in that the preliminary bleaching with hypochlorite is carried out with up to about 4 % by weight NaOCl and preferably with about 0.1 to 3 % by weight NaOCl, based on washing-active substance, the hypochlorite being added to the material to be bleached, preferably in several steps.

4. A process as claimed in Claims 1 to 3, characterized in that the elimination of excess hypochlorite on completion of the first bleaching step is effected by pH reduction into the mildly acidic range of from about 3.5 to below 7 and/or by addition of reducing agents, formaldehyde, alkali sulfites or alkali thiosulfates preferably being used as reducing agents.

5. A process as claimed in Claims 1 to 4, characterized in that sulfuric acid is used for pH adjustment during the peroxide bleaching step.

6. A process as claimed in Claims 1 to 5, characterized in that the hydrogen peroxide is used in quantities of up to 2.5 % by weight and preferably in quantities of 1 to 2 % by weight, expressed as 100 % $H_2O_2$ and based on washing-active substance.

7. A process as claimed in Claims 1 to 6, characterized in that approximately 10 to 15 % by weight NaOCl solutions and approximately 20 to 70 % by weight $H_2O_2$ solutions are used.

8. A process as claimed in Claims 1 to 7, characterized in that the hypochlorite bleaching step is carried out at temperatures of from about 50 to 75 °C and more especially at temperatures of from 55 to 70 °C, preferably over a period of 1 to 10 hours and more preferably over a period of 1 to 3 hours.

9. A process as claimed in Claims 1 to 8, characterized in that the intermediate reduction step and the subsequent bleaching step are carried out at temperatures above 50 °C and more especially at temperatures in the range from 55 to 70 °C.

10. A process as claimed in Claims 1 to 9, characterized in that all the steps of the process are carried out under normal pressure.

11. A process as claimed in Claims 1 to 10, characterized in that the aqueous salt pastes contain approximately 20 to 60 % by weight of alkali salts of the α-sulfofatty acid esters.

**Revendications**

1. Procédé de production d'esters actifs au lavage, de couleur stable, formant des pâtes salines aqueuses de couleur claire, dérivés d'acides gras α-sulfoniques et d'alcools en $C_1$ à $C_8$ monovalents (esters d'acides gras α-sulfoniques), en particulier à base de graisses ou d'huiles d'origine végétale et/ou animale et leurs mélanges avec des benzènes sulfonates d'alcoyle actifs au lavage au moyen de combinaisons de blanchiment d'un produit de départ brut de coloration foncée avec le peroxyde d'hydrogène et un hypochlorite de métal alcalin, caractérisé en ce que l'on transforme un produit de départ foncé par formation d'un sel de groupe α-sulfonique en une pâte d'ester de sulfonate, soumet la pâte d'ester sulfonate en milieu aqueux dans une zone de pH d'environ 7 à 11, de préférence de 7,5 à 10, d'abord à l'action d'un hypochlorite en vue d'un pré-mélange de blanchiment et finalement — en cas de besoin — élimine encore l'hypochlorite présent, à partir de quoi on blanchit à nouveau la pâte de sels dans la zone de pH d'environ 3,5 jusqu'à en dessous de 7, de préférence dans la zone de pH d'environ 4,0 à 6,5 avec du peroxyde d'hydrogène ou des substances qui fournissent du peroxyde d'hydrogène.

2. Procédé selon la revendication 1, caractérisé en ce que le pré-blanchiment à l'hypochlorite est interrompu environ au moment de l'optimum de blanchiment.

3. Procédé selon les revendications 1 et 2, caractérisé en ce que le pré-blanchiment par l'hypochlorite est effectué avec jusqu'à environ 4 % en poids d'hypochlorite de sodium, de préférence d'environ 0,1 à 3 % en poids d'hypochlorite de sodium respectivement calculé par rapport à la substance active de lavage, ou l'hypochlorite est ajouté de préférence en plusieurs étapes au produit à blanchir.

4. Procédé selon les revendications 1 à 3, caractérisé en ce que l'élimination de l'excès d'hypochlorite après achèvement du premier stade de blanchiment s'effectue par abaissement du pH dans la zone faiblement acide d'environ 3,5 jusqu'en dessous de 7 et/ou par addition d'un agent réducteur, procédé dans lequel comme agent réducteur, on met en œuvre de préférence le formaldéhyde, un sulfite alcalin ou un thiosulfate alcalin.

5. Procédé selon les revendications 1 à 4, caractérisé en ce que pour l'ajustement du pH lors du blanchiment par le peroxyde, on met en œuvre de l'acide sulfurique.

6. Procédé selon les revendications 1 à 5, caractérisé en ce que l'on met en œuvre le peroxyde d'hydrogène en quantités allant jusqu'à 2,5 % en poids, de préférence dans la zone de 1 à 2 % en poids, chaque fois calculé en tant que peroxyde d'hydrogène ($H_2O_2$) à 100 % et rapporté à la substance active de lavage.

7. Procédé selon les revendications 1 à 6, caractérisé en ce que l'on met en œuvre des solutions d'hypochlorite de sodium (ClONa) à environ 10-15 % et également des solutions de peroxyde d'hydrogène ($H_2O_2$) à environ 20-70 %.

8. Procédé selon les revendications 1 à 7, caractérisé en ce que le blanchiment par l'hypochlorite est effectué à des températures d'environ 50-75°, en particulier dans la zone de 55-70°, pour lesquelles de préférence on travaille avec une durée de blanchiment dans la zone de 1 à 10 h, en particulier dans la zone de 1 à 3 heures.

9. Procédé selon les revendications 1 à 8, caractérisé en ce que le stade intermédiaire de réduction et le blanchiment subséquent avec de l'eau oxygénée sont effectués à des températures au-dessus de 50°, en particulier dans la zone de 55-70°.

10. Procédé selon les revendications 1 à 9, caractérisé en ce qu'on a opéré pour toutes les étapes du procédé à la pression normale.

11. Procédé selon les revendications 1 à 10, caractérisé en ce qu'on a opéré avec des pâtes salines aqueuses d'une teneur en sels alcalins d'esters d'acide gras α-sulfonique d'environ 20 à 60 % en poids.